**Europäisches Patentamt**

**European Patent Office**

**Office européen des brevets**

(11) Veröffentlichungsnummer: **0 598 945 A1**

# EUROPÄISCHE PATENTANMELDUNG

(21) Anmeldenummer: **92120269.3**

(51) Int. Cl.5: **A61B 5/0408**

(22) Anmeldetag: **27.11.92**

(43) Veröffentlichungstag der Anmeldung:
**01.06.94 Patentblatt 94/22**

(84) Benannte Vertragsstaaten:
**AT BE CH DE ES FR GB IT LI NL SE**

(71) Anmelder: **FEHLING MEDICAL AG**
**Frankenstrasse 21**
**D-63791 Karlstein(DE)**

(72) Erfinder: **Krenzke, Gerhard, Dipl.-Ing. Dr. Ing.**
**Warschauer Strasse 77**
**W-1034 Berlin(DE)**

(74) Vertreter: **Patentanwälte Dipl.-Ing. Klaus**
**Westphal Dr.rer.nat. Otto Buchner Dr.rer.nat.**
**Bernd Mussgnug Dr.-Ing. Peter Neunert**
**Waldstrasse 33**
**D-78048 Villingen-Schwenningen (DE)**

(54) **Auswertungsverfahren zum EKG-Mapping.**

(57) Die Erfindung betrifft das EKG-Mapping mit mehreren auf den Thorax des Patienten auflegbaren Elektroden, deren Abstand vom Herz bekannt ist, wobei aus den Spannungen an den Elektroden zeitabhängige Größen berechnet werden, die die Multipolkomponenten des elektrischen Herzfeldes besonders hervorheben. Die Auswertung erfolgt durch folgende Schritte:
- aus den Elektrodenspannungen werden mit Hilfe der gemessenen Elektrodenabstände vom elektrischen Herzmittelpunkt, die auf eine Kugel, deren Mittelpunkt mit dem elektrischen Herzmittelpunkt übereinstimmt, projizierten Spannung berechnet,
- die projizierten Spannungen werden mit Korrekturfaktoren so korrigiert, daß der Einfluß der Leitfähigkeitsstruktur im Thorax und die Wanderung des elektrischen Herzmittelpunktes berücksichtigt werden,
- diese korrigierten Spannungen werden in eine Größe umgewandelt, deren Werte, unter der Annahme eines Dipolfeldes, über der Kugeloberfläche als konstant angenommen sind,
- die Differenzen dieser Größe zum Mittelwert werden über der Kugeloberfläche berechnet und
- diese Differenzen werden als Isointensitätslinien auf einer Kugel dargestellt.

Fig. 1

EP 0 598 945 A1

Die Erfindung geht aus von einem Auswertungsverfahren zum EKG-Mapping, bei dem mehrere auf den Thorax des Patienten auflegbare Elektroden, deren Abstand vom Herz bekannt ist, benutzt sind und bei dem aus den Spannungen an den Elektroden zeitabhängige Größen berechnet sind, die die Multipolkomponenten des elektrischen Herzfeldes besonders hervorheben.

In der kardiologischen Diagnostik wird zur Darstellung von Herzpotentialen das sogenannte EKG-Mapping verwendet, bei welchem eine Vielzahl von Elektroden zur Erfassung der Herzpotentiale auf der Thoraxoberfläche angeordnet ist. Dabei ist bekannt, die mit den Elektroden aufgenommenen Herzpotentiale auf der flächenhaft abgewickelten Thoraxoberfläche darzustellen ( H.Abel: Electrocardiology 19888, Excerpta Medica, Amsterdam 1989, S. 357-360 ). Dieses Verfahren hat den Nachteil, daß es die unterschiedlichen Abstände der Ableitungselektroden vom elektrischen Herzmittelpunkt und deren Einfluß auf das elektrische Feld nicht berücksichtigt.

Um den Einfluß der Thoraxkonfiguration auf das auf der Thoraxoberfläche gemessene Potentialfeld zu berücksichtigen, wird aus diesem Potentialfeld das Potentialfeld auf der Herzoberfläche berechnet. Diese Verfahren sind als Lösungen des "inversen Problems" bekannt. Entweder wird eine Modelloberfläche des Herzens angenommen ( IEEE Transactions on Biomedical Engineering Bd. 31 (1984), Nr.9, S. 611-621 ) oder die Herzform kann mit Hilfe eines NMR-Tomographen ( nuclear magnetic resonance ) ermittelt werden.

Anstelle des Potentialfeldes als Intensitätsfeld werden auch Isochronen auf der Herzoberfläche sowohl für Modellherzen (IEEE Transactions on Biomedical Engineering, Bd.31 (1984), Nr. 10, S. 652-659) wie auch für mit dem Tomographen gemessene Herzformen (IEEE Transactions on Biomedical Engineering, Bd.35 (1988), Nr.12, S. 1047-1058) dargestellt.

Die Berechnung der Potentialverteilung bzw. von Isochronen auf der Oberfläche einer theoretischen Herzform kann zu erheblichen Fehlinterpretationen führen, wenn Form und Lage des Modellherzens vom wirklichen Herzen abweichen. Dieses Verfahren ist nur für theoretische Untersuchungen geeignet. Die Messung der wirklichen Herzform mit dem NMR-Thomographen kann für Patienten mit metallischen Implantaten, wie Schrittmacherpatienten und herztransplantierte Patienten mit IMEK-Elektroden, lebensbedrohlich sein. Bei diesen Patienten kann die wirkliche Herzform und -lage auf diese Weise nicht ermittelt werden. Bei den anderen Patientengruppen ergeben sich in der Routine erhebliche Schwierigkeiten der Kopplung der Bildübertragung vom Tomographen zum EKG-Mapping-Gerät .

Bekannt ist ein Vorschlag (Probleme der räumlichen Vektorkardiographie. Int. Coll. Stary Smocovec 1961. Verlag d. Slowakischen Akademie d. Wiss., Bratislava 1963, S. 43-54), die auf dem Thorax gemessenen EKG-Kurven auf eine Kugel um den elektrischen Herzmittelpunkt zu projizieren. Die Projektion erfolgt entlang der Ableitlinien Kugelmittelpunkt-Elektrode entsprechend der Dipolhypothese. Die zeichnerische Darstellung kompletter EKG-Kurven an den Projektionspunkten auf der Kugeloberfläche ist unübersichtlich, hat eine geringe Auflösung in Zeit und Amplitude, beinhaltet keine Datenreduktion für eine komprimierte Aussage und ist deswegen für eine Routinediagnostik ungeeignet.

Außerdem ist die Darstellung von EKG-Kurven, Äquipotentiallinien oder Isochronen, wie in den vorstehend beschriebenen Verfahren, unabhängig von der Darstellungsfläche, nicht geeignet, Störungsgrenzen von lokalen Störungen der Feldausbreitung besonders zu markieren, weil lokale Amplituden immer im Verhältnis zum Gesamterregungszustand bewertet werden müssen. Am Meßort unter der Elektrode ist das Potential immer ein Summenpotential, erzeugt von allen erregten Orten im Herzen. Lokal begrenzte Störungen, die sich besonders in den höheren Multipolkomponenten darstellen, werden also unterdrückt.

Um die höheren Multipolkomponenten gesondert darzustellen, ist ein Vorschlag bekannt (Electrocardiology '87, Akademie-Verlag Berlin 1988, S. 165-167), nach dem von der Meßspannung an einem Elektrodenort die dorthin projizierten Spannungen des Frankschen Spannungsvektors abgezogen werden. Die Projektion des Frankschen Spannungsvektors wird mit Hilfe einer Transformationsmatrix, die statistisch aus EKG-Kurvenvergleichen gewonnen wurde, vorgenommen. Verfälschend wirkt, daß unabhängig von der Thoraxkonfiguration der Probanden die gleiche Matrix verwendet wird. Da für die Meßspannung die Thoraxform ebenfalls weder individuell noch im Mittel berücksichtigt wird, ist die Differenz zwischen der Meßspannung und der Projektion des Frankschen Spannungsvektors auch für ein Dipolfeld nicht konstant und der Wirkung höherer Multipole ist der Einfluß der Thoraxkonfiguration überlagert, was zu starken Verzerrungen der Isopotentiallinien führt und Vergleiche zwischen Patienten erschwert.

Der Erfindung liegt demgemäß die Aufgabe zugrunde, ein Verfahren der eingangs genannten Art zur Verfügung zu stellen, mit dem es möglich ist, Ausdehnung und Lage lokaler Feldverteilungen und die Ausbreitung des elektrischen Herzfeldes ohne Verfälschungen durch die Thoraxkonfiguration normiert darzustellen. ohne die Herzform und -lage genau zu kennen.

Die Aufgabe ist erfindungsgemäß bei dem eingangs genannten Verfahren dadurch gelöst, daß folgende Verfahrensvorschriften gegeben sind,

- aus den Elektrodenspannungen mit Hilfe der gemessenen Elektrodenabstände vom elektrischen Herzmittelpunkt, die auf eine Kugel, deren Mittelpunkt mit dem elektrischen Herzmittelpunkt übereinstimmt, projizierten Spannungen zu berechnen und

- die projizierten Spannungen mit Korrekturfaktoren so zu korrigieren, daß der Einfluß der Leitfähigkeitsstruktur im Thorax und die Wanderung des elektrischen Herzmittelpunktes berücksichtigt wird und

- diese korrigierten Spannungen in eine Größe umzuwandeln, deren Werte, unter der Annahme eines Dipolfeldes, über der Kugeloberfläche konstant sind und

- die Differenzen dieser Größe zum Mittelwert über der Kugeloberfläche zu berechnen und

- als Isointensitätslinien auf einer Kugel darzustellen. Vorteilhafte Ausführungsformen der Erfindung sind in den Unteransprüchen angegeben.

Das erfindungsgemäße Verfahren ist nachfolgend mit folgenden Zeichnugen an Ausführungsbeispielen erläutert.

Es stellen dar:

Fig. 1     einen Schnitt durch eine Ableitungsvorrichtung mit der schematischen Anordnung der Elektroden

Fig. 2     die Projektionskugel mit den projizierten Elektrodenpunkten, Vorderseite und Rückseite

Fig. 3     die Flächenaufteilung der den Elektroden zugeordneten Flächen auf der Kugelvorderseite

Fig. 4     die Isointensitätsliniendarstellung des Differenz-Map I bei einer Herzrejektion nach Herztransplantation

Fig. 5     die Isointensitätsliniendarstellung des Differenz-Map II bei einem Aneurisma

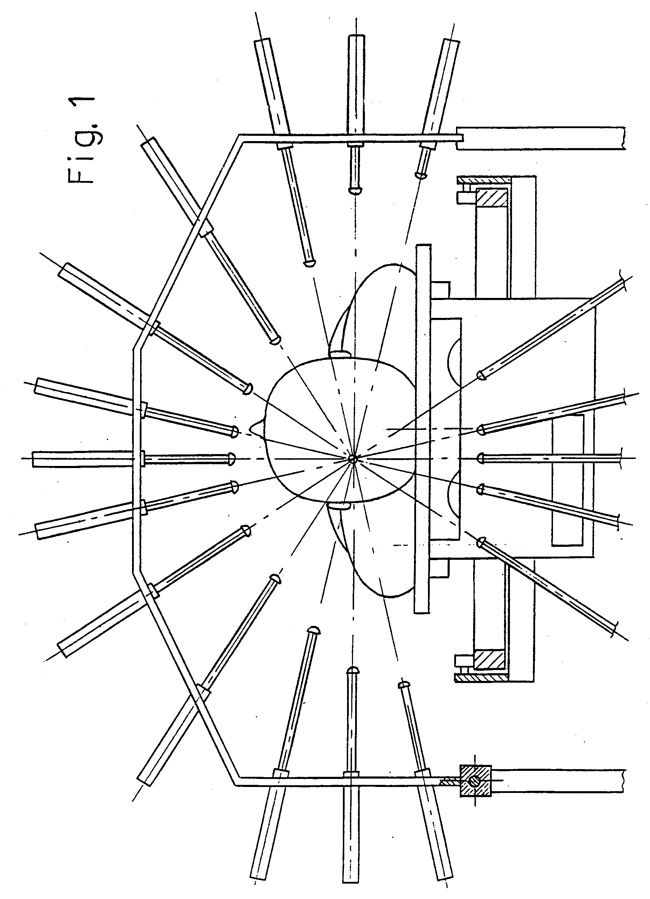

Im ersten Schritt werden mit einer Vorrichtung, von der in Fig.1 ein Schnitt schematisiert dargestellt ist, z.B. entsprechend DD 225 333 A1 an einer Vielzahl von Stellen auf dem Thorax des Patienten Spannungen gegenüber einem elektrischen Bezugspunkt, z.B. "central terminal" nach Wilson, gemessen. Außerdem wird der Abstand der Elektroden an den Meßstellen vom elektrischen Herzmittelpunkt ermittelt, wobei der elektrische Hersmittelpunkt mit einem Vorrichtungsbezugspunkt zur Deckung gebracht wird, nachdem die Lage des elektrischen Herzmittelpunktes im Thorax des Patienten in bekannter Weise (Circulation XIII (1956), H.5, S. 737-749) bestimmt wurde.

Aus der an einer Elektrode im Abstand $r_m$ vom Herzmittelpunkt auf dem Thorax gemessenen Spannung $U_m$ ergibt sich die auf eine Kugel mit dem Radius $r_k$ , zweckmäßigerweise gleich 10 cm gesetzt, um den Herzmittelpunkt als Kugelmittelpunkt projizierte und korrigierte Spannung

$$(1) \qquad K_m = a_m \frac{r_m^2 \, U_m}{r_k^2}$$

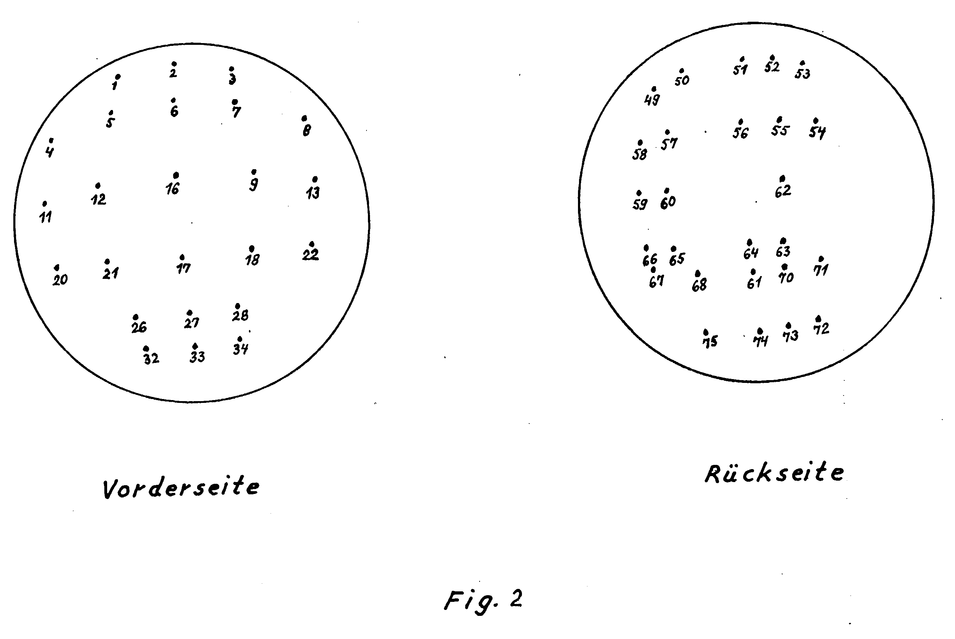

Die Projektionspunkte für eine Ableitanlage entsprechend Fig.1 sind in Fig.2 auf der Vorder- und Rückseite der Projektionskugel dargestellt. $a_m$ ist ein empirisch ermittelter Korrekturfaktor.

Die Gleichung (1) geht vom Dipolmodell aus. Bekannt ist (American Heart Journal Bd. 49 (1955), S. 670-681), daß bei den "Spiegeluntersuchungen" zur Bestimmung des elektrischen Herzmittelpunktes nicht ein Punkt, sondern ein Körper gefunden wird, in dem dieser Punkt definiert werden muß. Bei den "Spiegeluntersuchungen" wird üblicherweise der gesamte QRS-Komplex an den spiegelbildlichen Ableitstellen auf deckungsgleiche Kurvenform verglichen. Betrachtet man die Spannungen zu konkreten Zeitpunkten, so stellt man fest (Circulation Research Bd. 46 (1980), S. 415-425), daß der Dipolort außerdem wandert. Die Wanderung des Dipolortes ist eine der Ursachen für die Ausdehnung des Beschreibungspörpers des Dipolortes. Eine andere Ursache liegt in der Leitfähigkeitsinhomogenität des das Herz umgebenden Mediums. Diese Einflußfaktoren auf das Dipolfeld können mit dem Korrekturfaktor $a_m$ ausgeglichen werden. Die Korrekturfaktoren an den Elektrodenorten werden so gewählt, daß im Mittel für herzgesunde Probanden die korrigierte Feldverteilung auf der Kugeloberfläche einem Dipolfeld entspricht. Während des QRS-Komplexes werden alle 2 ms der Ort und die Größe der Maximal- und der Minimalamplitude auf der Kugeloberfläche ermittelt. Die Minimalwerte werden mit einem solchen Korrekturfaktor versehen, daß das Produkt gleich dem Maximalwert ist. Interpoliert man zwischen diesen Meßpunkten Ort und Größe des Korrekturfaktors, so erhält man geschlossene Raumkurven auf der Kugeloberfläche, entlang denen sich der Korrekturfaktor kontinuierlich verändert. Nun werden zwei Elektrodenprojektionspunkte auf der Kugel mit einem Großkreis verbunden. Die Großkreise zwischen den Elektrodenprojektionspunkten werden von einer unterschiedlichen Anzahl von Raumkurven verschiedener Probanden geschnitten. Die Korrekturfaktoren der Raumkurven an den Schnittstellen sind unterschiedlich groß. Mit Hilfe der linearen Regression dieser Schnittstellenkorrekturfaktoren werden die extrapolierten Korrekturfaktoren an den Elektrodenorten ermittelt. Ergeben sich an einem Elektrodenort in Verbindung mit den benachbarten Elektrodenorten unterschiedliche Korrekturfaktoren, so werden diese gemittelt. Es gibt nun benachbarte Elektrodenorte, zwischen denen deren Großkreise von keiner Raumkurve geschnitten werden. Diesen Elektrodenorten wird der Mittelwert der Korrekturfaktoren der beiden dichtest gelegenen Elektrodenorte mit Korrekturfaktoren zugeordnet.

Für die. Berechnung des Differenz-Map I wird nun davon ausgegangen, daß es eine Meßgröße für das Dipolfeld gibt, nämlich das Frank-EKG. Das Franksche EKG kann deswegen in Näherung als Maß für das Dipolfeld genommen werden, weil die Zusammenschaltung mehrerer Elektrodem über das Frank-Netzwerk eine gewisse Mittelwertbildung erzeugt, die kleinere Störungen des elektrischen Feldes unterdrückt, wie es auch aus Testungen (H.Semmler: Die klinische Wertigkeit des korrigierten orthogonalen Ruhe-EKG nach Frank im Vergleich zur konventionellen Elektrokardiographie in Ruhe für die Erfassung der Koronarartherosklerose. Dissertation B, Humboldt-Universität Berlin 1979) bekannt ist.

Der Spannungsvektor nach Frank wird aus den drei Ableitungen X,Y,Z des Frank-EKG, das zusammen mit den Mapping-Ableitungen registriert wird, bestimmt. Die Projektion des Spannungsvektors auf die Ableitachse vom Kugelmittelpunkt zur m-ten Elektrode mit den Koordinaten $R_m$, $S_m$, $T_m$ ergibt

$$(2) \qquad F_m = \frac{X\,R_m + Y\,S_m + Z\,T_m}{\sqrt{R_m^2 + S_m^2 + T_m^2}}$$

Diese Projektionen des Spannungsvektors entsprechen Meßwerten auf einer Kugeloberfläche mit dem unbekannten Radius $r_y$. Dieser Radius ergibt sich daraus, daß im Frank-System die Werte der Y-Ableitung nicht korrigiert werden, also $r_y$ etwa dem Abstand vom Herzmittelpunkt bis zur linken Leistenbeuge entspricht. Die projizierten Spannungen des Frank-Systems verhalten sich zu den korrigierten Elektroden-spannungen wie $r_k^2/r_y^2$. Die Differenz beider Spannungen

$$(3) \qquad D_m = K_m - F_m$$

müßte also konstant über der ganzen Kugeloberfläche sein. Um auch kleine Störungen feststellen zu können, werden die Differenzen $D_m$ über alle Elektroden gemittelt.

$$(4) \qquad M = \frac{1}{n} \sum_{\mu=1}^{n} A_\mu\, D_\mu$$

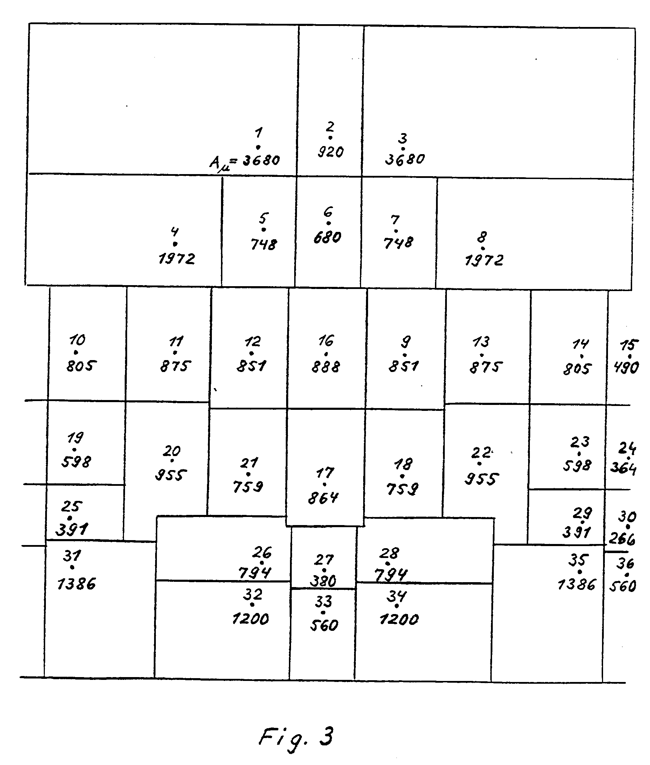

$A_\mu$ ist ein korrigierender Flächenanteil auf der Kugeloberfläche. Die jeweilige $\mu$-te Elektrode liegt dabei in definierter Weise innerhalb des zugeordneten korrigierenden Flächenanteils $A_\mu$. In Fig.3 ist für die vordere Halbkugel der Fig.2 die Anordnung der korrigierenden Flächenanteile dargestellt. Die Rechtecke werden so konstruiert, daß die Elektrodenpunkte in benachbarten Rechtecken etwa gleich weit von der Trennlinie dieser beiden Rechtecke entfernt sind. Die Summe sämtlicher korrigierender Flächenanteile ergibt den Flächeninhalt der gesamten Projektionsoberfläche d.h. der Kugel. $A_M$ ist der Mittelwert sämtlicher Flächen-anteile $A_\mu$. Es werden nun die Differenzen

$$(5) \qquad Z_m = |M - A_M\, D_m|$$

für alle n Elektroden ermittelt. Die Werte $Z_m$ werden der Größe nach geordnet und die p Werte $D_m$ (z.B. p = 5), die zu den p größten Werten $Z_m$ gehören, werden aus der Berechnung eines korrigierten Mittelwertes

4

$$(6) \qquad M_k = \frac{1}{n-p} \sum_{\mu=p+1}^{n} A_\mu \, D_\mu$$

ausgeschlossen.

Als Differenz-Map I werden die korrigierten Differenzen

$$(7) \qquad W_m = M_k - A_M \, D_m$$

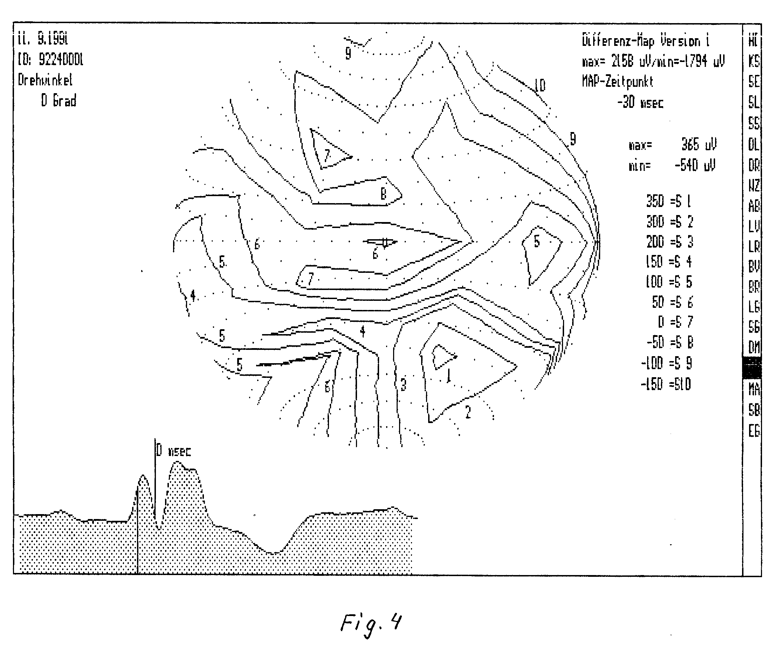

als Isointensitätslinien dargestellt. Die Darstellung, siehe Fig.4, erfolgt für einen ausgewählten Zeitpunkt innerhalb des EKG-Zyklus. Die Isointensitätslinien aufeinander folgender Zeitpunkte werden so schnell dargeboten, daß auf der Kugeloberfläche das Wandern dieser Linien in einem für das Auge verfolgbaren Tempo zeitverzögert gegenüber dem normalen Erregungsablauf erfolgt. In der Fig.4 ist die Kugel um den elektrischen Herzmittelpunkt in frontaler Ansicht dargestellt. Die untere Hälfte der Figur zeigt eine der 75 EKG-Ableitungen, die für die Berechnung des Differenz-Map zu dem markierten Zeitpunkt verwendet wurde. Zu diesem Zeitpunkt fehlt die übliche Erregung der rechten Herzkammer. Dies Ischämiekriterium ist ein deutliches Zeichen für die vorliegende Abstoßungsreaktion bei einem herztransplantierten Patienten.

Eine weitere aus dem elektrischen Feld des Herzens abgeleitete Größe wird in dem Differenz-Map II in Form von Isointensitätslinien graphisch dargestellt. In diesem Differenz-Map II wird für jede Elektrode m aus der korrigierten Spannungsamplitude eine normiertr Differenz berechnet gemäß der Gleichung

$$(8) \qquad D_m = \frac{A_M \left| \dfrac{K_m}{\cos \alpha_m} \right| - \dfrac{1}{n} \sum_{\mu=1}^{n} A_\mu \left| \dfrac{K_\mu}{\cos \alpha_\mu} \right|}{\dfrac{1}{n} \sum_{\mu=1}^{n} A_\mu \left| \dfrac{K_\mu}{\cos \alpha_\mu} \right|}$$

Der Winkel $\alpha_\mu$ ist dabei aus den drei gemessenen Ableitungen X,Y,Z des Frankschen EKG und den Komponenten des Ortsvektors R,S,T, der $\mu$-ten Elektrode zu berechnen gemäß der Gleichung

$$(9) \qquad \cos \alpha_\mu = \frac{X \, R_\mu + Y \, S_\mu + Z \, T_\mu}{\sqrt{R_\mu^2 + S_\mu^2 + T_\mu^2} \ \sqrt{X^2 + Y^2 + Z^2}}$$

Die unter idealen Bedingungen über der Kugeloberfläche konstanten Größen in Gleichung (8) sind $K_m/\cos \alpha_m$ bzw. $K_\mu/\cos \alpha_\mu$ .

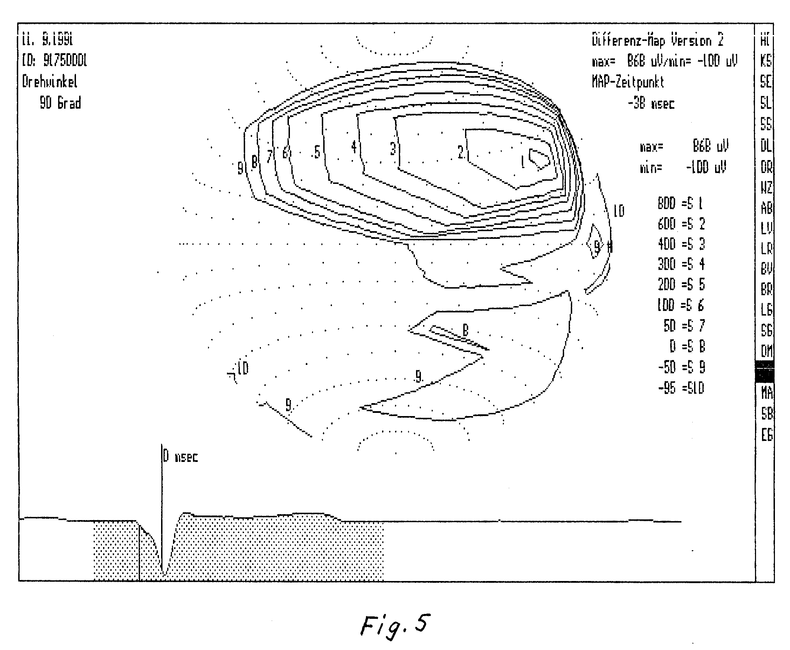

Das Differenz-Map II kann als Maß für die Abweichung der Leitfähigkeit in dem Raumwinkel, den die zur Elektrode gehörenden Flächenanteile einnehmen, von der mittleren, als homogen verteilt angenommenen Leitfähigkeit angesehen werden. Es eignet sich insbesondere zur Darstellung von Ausdehnungsgrenzen leitungsgestörter Areale z.B. beim Infarkt oder Aneurisma entsprechend Fig.5. Die Kugel um den elektrischen Herzmittelpunkt ist vom Betrachter aus um 90 Grad linksherum gedreht, so daß man in Richtung auf die linke Lateralwand des Herzens schaut. Um den hoch sitzenden Posterolateralinfarkt erstreckt sich nach vorn das deutlich begrenzte Gebiet des Aneurismas.

Liste der Ausgangsgrößen und berechneten Größen.

$U_m, U_\mu$ $\qquad$ = Spannung an der Elektrode m bzw. $\mu$

$a_m, a_\mu$ $\qquad$ = Korrekturfaktor für die Spannung an der Elektrode m bzw. $\mu$

| | | |
|---|---|---|
| X, Y, Z | = | Koordinaten des Frank-Vektors |
| $R_m$, $S_m$, $T_m$ | = | Koordinaten der Elektrode m |
| $R_\mu$, $S_\mu$, $T_\mu$ | = | Koordinaten der Elektrode $\mu$ |
| $A_\mu$ | = | Teilfläche einer Kugeloberfläche |
| $A_M$ | = | Mittelwert aller Teilflächen einer Kugeloberfläche |
| $r_m$, $r_\mu$ | = | Abstand der Elektrode m bzw. $\mu$ vom elektrischen Herzmittelpunkt |
| $r_k$ | = | Radius einer Projektionskugel |
| $r_y$ | = | Radius der Projektionskugel des Frankschen Ableitungssystems |
| $\cos\alpha_m$, $\cos\alpha_\mu$ | = | Winkel zwischen Ableitachse zur Elektrode m bzw. $\mu$ und Frank-Vektor |
| $K_m$ | = | auf eine Kugel projizierte und korrigierte Spannung an der Elektrode m |
| $F_m$ | = | Projektion des Frankschen Spannungsvektors auf die Ableitachse der m.-ten Elektrode |
| $D_m$ | = | Spannungsdifferenz |
| M | = | Mittelwert von Spannungsdifferenzen |
| $Z_m$ | = | Mittelwertsabweichung einer Spannungsdifferenz |
| $M_k$ | = | korrigierter Mittelwert von Spannungsdifferenzen |
| $W_m$ | = | Differenz-Map I |
| $D_m$ | = | Differenz-Map II |

**Patentansprüche**

1. Auswertungsverfahren zum EKG-Mapping, bei dem mehrere auf den Thorax des Patienten auflegbare Elektroden, deren Abstand vom Herz bekannt ist, benutzt sind und bei dem aus den Spannungen an den Elektroden zeitabhängige Größen berechnet sind, die die Multipolkomponenten des elektrischen Herzfeldes besonders hervorheben, dadurch gekennzeichnet, daß folgende Verfahrensvorschriften gegeben sind,
   - aus den Elektrodenspannungen mit Hilfe der gemessenen Elektrodenabstände vom elektrischen Herzmittelpunkt, die auf eine Kugel, deren Mittelpunkt mit dem elektrischen Herzmittelpunkt übereinstimmt, projizierten Spannungen zu berechnen und
   - die projizierten Spannungen mit Korrekturfaktoren so zu korrigieren, daß der Einfluß der Leitfähigkeitsstruktur im Thorax und die Wanderung des elektrischen Herzmittelpunktes berücksichtigt wird und
   - diese korrigierten Spannungen in eine Größe umzuwandeln, deren Werte, unter der Annahme eines Dipolfeldes, über der Kugeloberfläche als konstant angenommen sind und
   - die Differenzen dieser Größe zum Mittelwert über der Kugeloberfläche zu berechnen und
   - diese Differenzen als Isointensitätslinien auf einer Kugel darzustellen.

2. Verfahren nach Anspruch 1 dadurch gekennzeichnet, daß als über der Kugeloberfläche der Kugel mit dem Radius $r_k$ als konstant angenommene Größe an jeder Elektrode m

$$D_m = a_m \frac{U_m\, r_m^2}{r_k^2} - F_m$$

aus der an der Elektrode m gemessenen Spannung $U_m$, der Projektion $F_m$ des aus dem Frank-EKG berechneten Spannungsvektors auf die durch den elektrischen Herzmittelpunkt und den Ort der Elektrode m definierten Ableitachse, dem Abstand $r_m$ der Elektrode m vom elektrischen Herzmittelpunkt und einem empirisch ermittelten Korrekturfaktor $a_m$, der den Einfluß der Leitfähigkeitsstruktur im Thorax und die Wanderung des elektrischen Herzmittelpunktes berücksichtigt, bestimmt wird und daß die aus der Größe $D_m$ abgeleitete und auf der Kugeloberfläche in Form von Isointensitätslinien dargestellte Differenz

$$W_m = [\ \frac{1}{n-p}\ \sum_{\mu=p+1}^{n} A_\mu\ D_\mu\ ]\ -\ A_M\ D_m$$

ist, mit $A_\mu$ als wichtender Flächenanteil auf der Projektionsoberfläche, der die unterschiedlichen gegenseitigen Abstände der Elektroden untereinander berücksichtigt und $A_M$ als Mittelwert aller $A_\mu$, wobei die p von den n Größen $D_\mu$ für die Berechnung nicht verwendet werden, die zu den p größten Werten der Differenzen

$$Z_m = |\ [\ \frac{1}{n}\ \sum_{\mu=1}^{n} A_\mu\ D_\mu\ ]\ -\ A_M\ D_m\ |$$

gehören.

3. Verfahren nach Anspruch 1 dadurch gekennzeichnet, daß die auf der Kugeloberfläche mit dem Kugelradius $r_k$ als konstant angenomene Größe

$$\frac{a_m\ r_m^2\ U_m}{r_k^2\ \cos\alpha_m}$$

bzw.

$$\frac{a_\mu\ r_\mu^2\ U_\mu}{r_k^2\ \cos\alpha_\mu}$$

ist und in Form von Isointensitätslinien die normierte Differenz

$$D_m = \frac{A_M\ \left|\dfrac{a_m\ r_m^2\ U_m}{\cos\alpha_m}\right|\ -\ \dfrac{1}{n}\ \sum_{\mu=1}^{n} A_\mu\ \left|\dfrac{a_\mu\ r_\mu^2\ U_\mu}{\cos\alpha_\mu}\right|}{\dfrac{1}{n}\ \sum_{\mu=1}^{n} A_\mu\ \left|\dfrac{a_\mu\ r_\mu^2\ U_\mu}{\cos\alpha_\mu}\right|}$$

dargestellt wird, wobei $U_m$ bzw. $U_\mu$ die Spannungsamplituden an der m-ten bzw. $\mu$-ten Elektrode , $\alpha_m$ bzw $\alpha_\mu$ die Winkel zwischen der Ableitachse der m-ten bzw. $\mu$-ten Elektrode und dem Frankschen Spannungsvektor, $A_\mu$ ein wichtender Flächenanteil auf der Projektionsoberfläche, der die unterschiedlichen gegenseitigen Abstände der Elektroden untereinander berücksichtigt und $A_M$ der Mittelwert aller $A_\mu$, $r_m$ bzw. $r_\mu$ die Abstände der m-ten bzw. $\mu$-ten Elektrode vom elektrischen Herzmittelpunkt, $a_m$ bzw. $a_\mu$ empirisch ermittelte Korrekturfaktoren, die den Einfluß der Leitfähigkeitsstruktur im Thorax und die Wanderung des elektrischen Herzmittelpunktes berücksichtigen, sind und $\cos \alpha_m$ bzw. $\cos \alpha_\mu$ sich aus

$$\cos \alpha_\mu = \frac{X\,R_\mu + Y\,S_\mu + Z\,T_\mu}{\sqrt{R_\mu^2 + S_\mu^2 + T_\mu^2}\ \sqrt{X^2 + Y^2 + Z^2}}$$

mit den Koordinaten $\{R_\mu, S_\mu, T_\mu\}$ der $\mu$-ten Elektrode und den Komponenten $\{X, Y, Z\}$ des mit Hilfe des Frankschen Ableitungssystems gemessenen Spannungsvektors ergeben.

Fig. 1

Rückseite

Vorderseite

Fig. 2

Fig. 3

Fig. 4

Fig. 5

**Europäisches Patentamt**

# EUROPÄISCHER RECHERCHENBERICHT

Nummer der Anmeldung

EP 92 12 0269

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-3 718 071 (SLOVENSKA AKADEMIA VIED BRATISLAVA) <br> * das ganze Dokument * <br> --- | 1-3 | A61B5/0408 |
| A | WO-A-8 200 089 (STORVRETA SPORT AB) <br> * das ganze Dokument * <br> --- | 1-3 | |
| A | MEDICAL AND BIOLOGICAL ENGINEERING AND COMPUTING. <br> Bd. 21, Nr. 1, Januar 1983, STEVENAGE GB <br> Seiten 83 - 90 <br> K.YAJIMA ET AL. 'Body surface potential mapping system equipped with a microprocessor for the dynamic observation of potential patterns' <br> * das ganze Dokument * <br> --- | 1-3 | |
| D,A | IEEE TRANSACTIONS ON BIO-MEDICAL ENGINEERING <br> Bd. 35, Nr. 12, Dezember 1988, NEW YORK US <br> Seiten 1047 - 1058 <br> G.HUISKAMP ET AL. 'The Depolarization Sequence of the Human Heart Surface Computed from Measured Body Surface Potentials' <br> * das ganze Dokument * <br> --- | 1-3 | **RECHERCHIERTE SACHGEBIETE (Int. Cl.5)** <br><br> A61B |
| D,A | DD-A-225 333 (HUMBOLDT-UNIVERSITÄT ZU BERLIN) <br> * das ganze Dokument * <br><br> ----- | 1-3 | |

Der vorliegende Recherchenbericht wurde für alle Patentansprüche erstellt

| Recherchenort | Abschlußdatum der Recherche | Prüfer |
|---|---|---|
| DEN HAAG | 22 JULI 1993 | HUNT B.W. |